(19) Europäisches Patentamt
European Patent Office
Office européen des brevets

(11) Veröffentlichungsnummer: **0 580 042 A2**

# (12) EUROPÄISCHE PATENTANMELDUNG

(21) Anmeldenummer: **93111029.0**

(22) Anmeldetag: **09.07.93**

(51) Int. Cl.5: **C07D 487/04**, A01N 43/58

(30) Priorität: **22.07.92 DE 4224165**

(43) Veröffentlichungstag der Anmeldung: **26.01.94 Patentblatt 94/04**

(84) Benannte Vertragsstaaten: **BE CH DE ES FR GB IT LI NL**

(71) Anmelder: **BAYER AG**

**D-51368 Leverkusen(DE)**

(72) Erfinder: **Seitz, Thomas, Dr.**
**Mozartstrasse 32**
**D-40789 Monheim(DE)**
Erfinder: **Lürssen, Klaus, Dr.**
**August-Kierspel-Strasse 145**
**D-51469 Bergisch Gladbach(DE)**
Erfinder: **Santel, Hans-Joachim, Dr.**
**Grünstrasse 9a**
**D-51371 Leverkusen(DE)**
Erfinder: **Schmidt, Robert Rudolf**
**Im Waldwinkel 110**
**D-51467 Bergisch Gladbach(DE)**

(54) **Imidazolinyl-pyrrolo-pyridazine.**

(57) Die Erfindung betrifft neue Imidazolinyl-pyrrolo-pyridazine der allgemeinen Formel (I)

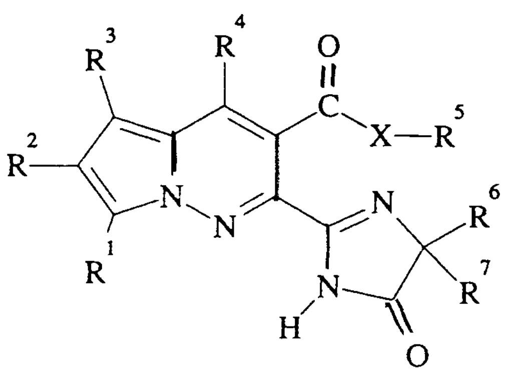

(I)

in welcher

$R^1$, $R^2$, $R^3$, $R^4$, $R^6$ und $R^7$ für Wasserstoff, Halogen, Alkyl, Halogenalkyl, Cycloalkyl oder für gegebenenfalls substituiertes Aryl stehen,

$R^5$ für Wasserstoff, für jeweils gegebenenfalls substituiertes Alkyl, Alkenyl, Alkinyl, Cycloalkyl, Cycloalkenyl, Aryl oder Heterocyclyl, für ein Äquivalent eines anorganischen oder organischen Kations oder für einen Rest der Formel

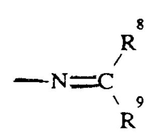

EP 0 580 042 A2

steht und

| | |
|---|---|
| X | für Sauerstoff oder Schwefel steht, wobei |
| $R^8$ | für Wasserstoff oder Alkyl steht und |
| $R^9$ | für Alkyl oder für gegebenenfalls substituiertes Aryl steht oder |
| $R^8$ und $R^9$ | gemeinsam mit dem Kohlenstoffatom, an welches sie gebunden sind, für einen Cycloalkylrest stehen, |

mehrere Verfahren zu ihrer Herstellung,
die neuen Zwischenprodukte der allgemeinen Formel (II)

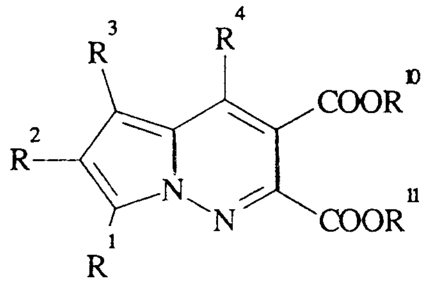

(II)

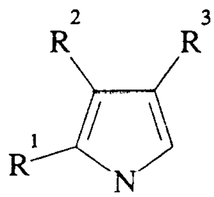

(VIII)

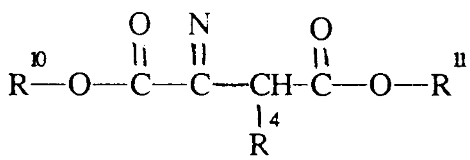

in welchen
$R^1$, $R^2$, $R^3$, $R^4$, $R^{10}$ und $R^{11}$ die oben angegebenen Bedeutungen haben,
sowie die Verwendung der neuen Imidazolinyl-pyrrolo-pyridazine der allgemeinen Formel (I) als Herbizide.

Die Erfindung betrifft neue Imidazolinyl-pyrrolo-pyridazine, mehrere Verfahren zu ihrer Herstellung, neue Zwischenprodukte sowie ihre Verwendung als Herbizide.

Es ist bekannt, daß bestimmte Imidazolinylpyridine wie beispielsweise die Verbindung 2-(4,4-Dimethylimidazolin-5-on-2-yl)-pyridin-3-carbonsäuremethylester herbizide Eigenschaften besitzen (vergl. z.B. EP 41623).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue Imidazolinyl-pyrrolo-pyridazine der allgemeinen Formel (I),

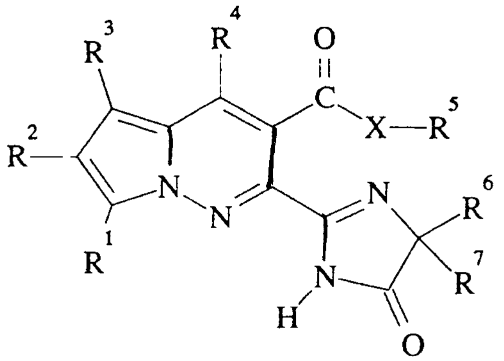

(I)

in welcher

| | |
|---|---|
| $R^1$, $R^2$, $R^3$, $R^4$, $R^6$ und $R^7$ | für Wasserstoff, Halogen, Alkyl, Halogenalkyl, Cycloalkyl oder für gegebenenfalls substituiertes Aryl stehen, |
| $R^5$ | für Wasserstoff, für jeweils gegebenenfalls substituiertes Alkyl, Alkenyl, Alkinyl, Cycloalkyl, Cycloalkenyl, Aryl oder Heterocyclyl, für ein Äquivalent eines anorganischen oder organischen Kations oder für einen Rest der Formel 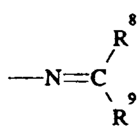 steht und |
| X | für Sauerstoff oder Schwefel steht, wobei |
| $R^8$ | für Wasserstoff oder Alkyl steht und |
| $R^9$ | für Alkyl oder für gegebenenfalls substituiertes Aryl steht oder |
| $R^8$ und $R^9$ | gemeinsam mit dem Kohlenstoffatomen, an welches sie gebunden sind, für einen Cycloalkylrest stehen, |

gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen Imidazolinyl-pyrrolo-pyridazine der allgemeinen Formel (I),

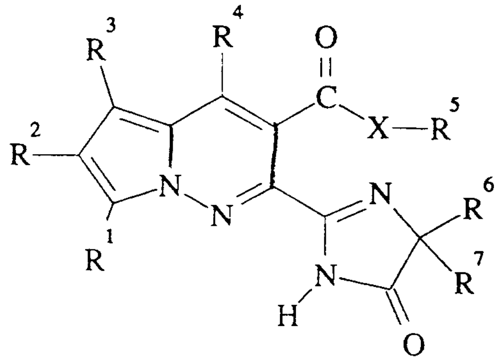

(I)

in welcher

$R^1$, $R^2$, $R^3$, $R^4$, $R^6$ und $R^7$ für Wasserstoff, Halogen, Alkyl, Halogenalkyl, Cycloalkyl oder für gegebenenfalls substituiertes Aryl stehen,

$R^5$ für Wasserstoff, für jeweils gegebenenfalls substituiertes Alkyl, Alkenyl, Alkinyl, Cycloalkyl, Cycloalkenyl, Aryl oder Heterocyclyl, für ein Äquivalent eines anorganischen oder organischen Kations oder für einen Rest der Formel

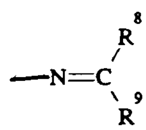

steht und

X für Sauerstoff oder Schwefel steht, wobei

$R^8$ für Wasserstoff oder Alkyl steht und

$R^9$ für Alkyl oder für gegebenenfalls substituiertes Aryl steht oder

$R^8$ und $R^9$ gemeinsam mit dem Kohlenstoffatom, an welches sie gebunden sind, für einen Cycloalkylrest stehen,

erhält, wenn man

(a) Pyrrolopyridazin-dicarbonsäurediester der Formel (II),

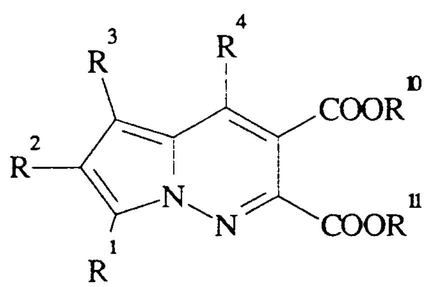

(II)

in welcher

$R^1$, $R^2$, $R^3$ und $R^4$ die oben angegebenen Bedeutungen haben und

$R^{10}$ und $R^{11}$ unabhängig voneinander jeweils für Alkyl stehen

mit $\alpha$-Aminosäureamiden der Formel (III),

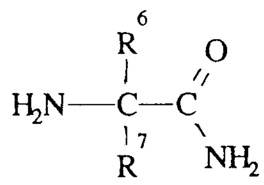

(III)

in welcher

$R^6$ und $R^7$ die oben angegebenen Bedeutungen haben,

gegebenenfalls in Gegenwart eines Verdünnungsmittels und gegebenenfalls in Gegenwart eines Reaktionshilfsmittels umsetzt und gegebenenfalls

(b) in einer anschließenden Folgereaktion die so erhältlichen Imidazolinyl-pyrrolopyridazin-carbonsäuren der Formel (Ia),

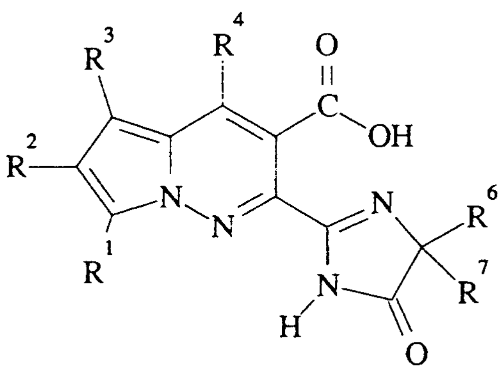

(Ia)

in welcher

$R^1$, $R^2$, $R^3$, $R^4$, $R^6$ und $R^7$ die oben angegebenen Bedeutungen haben,

zunächst in einer 1.Stufe mit einem Kondensationsmittel gegebenenfalls in Gegenwart eines Verdünnungsmittels und gegebenenfalls in Gegenwart eines Reaktionshilfsmittels umsetzt und anschließend die so erhältlichen Imidazo-pyrrolo-pyridazine der Formeln (IVa) und (IVb),

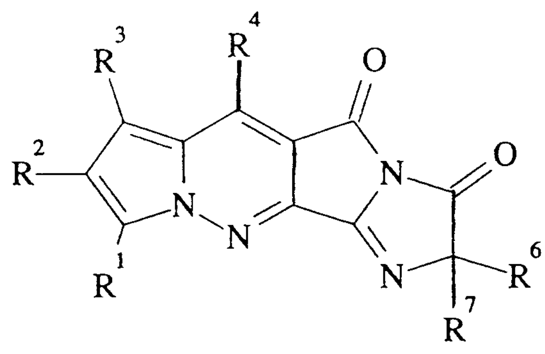

(IVa)

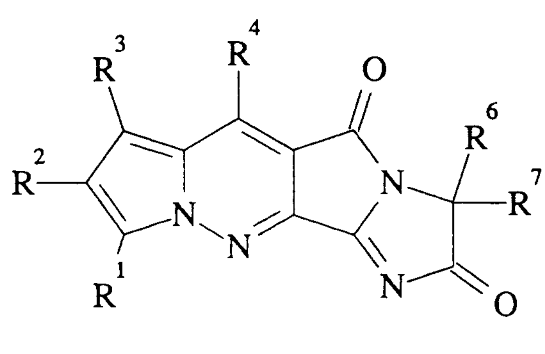

(IVb)

in welcher

$R^1$, $R^2$, $R^3$, $R^4$, $R^6$ und $R^7$ die oben angegebenen Bedeutungen haben,

in einer anschließenden 2. Stufe mit Alkoholen, Thiolen oder Oximen der Formel (V),

$R^5$-X-H (V)

in welcher

$R^5$ und X die oben angegebenen Bedeutungen haben,

gegebenenfalls in Gegenwart eines Verdünnungsmittels und gegebenenfalls in Gegenwart eines Reaktionshilfsmittels umsetzt.

Schließlich wurde gefunden, daß die neuen Imidazolinyl-pyrrolo-pyridazine der allgemeinen Formel (I) ebenso wie die als Zwischenprodukte benannten Imidazo-pyrrolo-pyridazine der Formeln (IVa) und (IVb) herbizide Eigenschaften besitzen. Überraschenderweise zeigen die erfindungsgemäßen Imidazolinyl-pyrrolo-pyridazine der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten Imidazolylpyridinen, wie beispielsweise die Verbindung 2-(4,4-Dimethylimidazolin-5-on-2-yl)-pyridin-3-carbonsäuremethylester, welche chemisch und wirkungsmäßig naheliegende Verbindungen sind.

Die erfindungsgemäßen Imidazolinyl-pyrrolo-pyridazine sind durch die Formel (I) allgemein definiert. Bevorzugt sind Verbindungen der Formel (I), bei welchen

$R^1$, $R^2$, $R^3$, $R^4$, $R^6$ und $R^7$ für Wasserstoff, Fluor, Chlor, Brom, Iod, für geradkettiges oder verzweigtes Alkyl mit 1 bis 8 Kohlenstoffatomen, Halogenalkyl mit 1 bis 8 Kohlenstoffatomen und 1 bis 17 gleichen oder verschiedenen Halogenatomen, Cycloalkyl mit 3 bis 8 Kohlenstoffatomen oder für gegebenenfalls einfach oder mehrfach, gleich oder verschieden substituiertes Phenyl stehen, wobei als Substituenten infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^5$ für Wasserstoff oder für ein Äquivalent eines Alkali-, Erdalkali- oder eines gegebenenfalls einfach oder mehrfach, gleich oder verschieden durch geradkettiges oder verzweigtes Alkyl mit 1 bis 18 Kohlenstoffatomen und/oder Benzyl substituierten Ammoniumkations steht,

außerdem für gegebenenfalls einfach oder mehrfach, gleich oder verschieden substituiertes, geradkettiges oder verzweigtes Alkyl mit 1 bis 18 Kohlenstoffatomen steht, wobei als Substituenten infrage kommen:

Fluor, Chlor, Brom, Iod, Hydroxy, Cyano, Nitro, Aminocarbonyl, Formamido, jeweils geradkettiges oder verzweigtes Alkoxy, Alkoxycarbonyl, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 8 Kohlenstoffatomen in den einzelnen Alkylteilen und gegebenenfalls 1 bis 17 gleichen oder verschiedenen Halogenatomen, jeweils gegebenenfalls im Aryl- oder Heterocyclylteil einfach oder mehrfach, gleich oder verschieden substituiertes Aryl, Aryloxy, Arylthio, Arylamino, Diarylamino, Arylcarbonyl oder Arylsulfonyl mit jeweils 6 bis 10 Kohlenstoffatomen in den einzelnen Arylteilen oder Heterocyclyl mit 2 bis 9 Kohlenstoffatomen und 1 bis 5 gleichen oder verschiedenen Heteroatomen, insbesondere Stickstoff, Sauerstoff und/oder Schwefel, wobei als Aryl- oder Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils

1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

außerdem für gegebenenfalls einfach oder mehrfach, gleich oder verschieden substituiertes, geradkettiges oder verzweigtes Alkenyl mit 2 bis 8 Kohlenstoffatomen steht, wobei als Substituenten infrage kommen:

Fluor, Chlor, Brom, Iod, Cyano oder gegebenenfalls einfach oder mehrfach, gleich oder verschieden substituiertes Aryl mit 6 bis 10 Kohlenstoffatomen, wobei als Substituenten infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;

außerdem für gegebenenfalls einfach oder mehrfach, gleich oder verschieden substituiertes, geradkettiges oder verzweigtes Alkinyl mit 2 bis 8 Kohlenstoffatomen steht, wobei als Substituenten infrage kommen:

Fluor, Chlor, Brom oder Iod;

außerdem für jeweils gegebenenfalls einfach oder mehrfach, gleich oder verschieden substituiertes Cycloalkyl oder Cycloalkenyl mit jeweils 3 bis 8 Kohlenstoffatomen steht, wobei als Substituenten jeweils infrage kommen:

Fluor, Chlor, Brom, Iod sowie jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen;

außerdem für jeweils gegebenenfalls einfach oder mehrfach, gleich oder verschieden substituiertes Aryl mit 6 bis 10 Kohlenstoffatomen oder Heterocyclyl mit 2 bis 9 Kohlenstoffatomen und 1 bis 5 gleichen oder verschiedenen Heteroatomen, insbesondere Stickstoff, Sauerstoff und/oder Schwefel steht, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

und außerdem für einen Rest der Formel

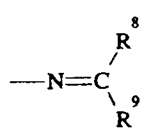

steht und

X für Sauerstoff oder Schwefel steht, wobei

$R^8$ für Wasserstoff oder Alkyl mit 1 bis 8 Kohlenstoffatomen steht und

$R^9$ für Alkyl mit 1 bis 8 Kohlenstoffatomen oder für gegebenenfalls einfach oder mehrfach, gleich oder verschieden substituiertes Phenyl steht, wobei als Substituenten infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges

oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl oder

$R^8$ und $R^9$ gemeinsam mit dem Kohlenstoffatom, an welches sie gebunden sind, für einen Cycloalkylrest mit 3 bis 8 Kohlenstoffatomen stehen.

Besonders bevorzugt sind Verbindungen der Formel (I), bei welchen

$R^1$, $R^2$, $R^3$, $R^4$, $R^6$ und $R^7$ für Wasserstoff, Fluor, Chlor, Brom, für geradkettiges oder verzweigtes Alkyl mit 1 bis 6 Kohlenstoffatomen, Halogenalkyl mit 1 bis 6 Kohlenstoffatomen und 1 bis 13 gleichen oder verschiedenen Halogenatomen, Cycloalkyl mit 3 bis 7 Kohlenstoffatomen oder für gegebenenfalls einfach bis dreifach, gleich oder verschieden substituiertes Phenyl stehen, wobei als Substituenten infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis dreifach, gleich oder verschieden durch Halogen und/oder geradkettiges oder verzweigtes Alkyl mit 1 bis 4 Kohlenstoffatomen substituiertes Phenyl;

$R^5$ für Wasserstoff oder für ein Äquivalent eines Alkali-, Erdalkali- oder eines gegebenenfalls einfach bis vierfach, gleich oder verschieden durch geradkettiges oder verzweigtes Alkyl mit 1 bis 16 Kohlenstoffatomen und/oder Benzyl substituierten Ammoniumkations steht,

außerdem für gegebenenfalls einfach bis dreifach, gleich oder verschieden substituiertes, geradkettiges oder verzweigtes Alkyl mit 1 bis 18 Kohlenstoffatomen steht, wobei als Substituenten infrage kommen:

Hydroxy, Cyano, Nitro, Aminocarbonyl, Formamido, jeweils geradkettiges oder verzweigtes Alkoxy, Alkoxycarbonyl, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkysulfonyl mit jeweils 1 bis 6 Kohlenstoffatomen in den einzelnen Ajkylteilen und gegebenenfalls 1 bis 13 gleichen oder verschiedenen Halogenatomen, jeweils gegebenenfalls im Aryl- oder Heterocyclylteil einfach bis dreifach, gleich oder verschieden substituiertes Aryl, Aryloxy, Arylthio, Arylamino. Diarylamino, Arylcarbonyl oder Arylsulfonyl mit jeweils 6 oder 10 Kohlenstoffatomen in den einzelnen Arylteilen oder Heterocyclyl mit 2 bis 9 Kohlenstoffatomen und 1 bis 4 gleichen oder verschiedenen Heteroatomen, insbesondere Stickstoff, Sauerstoff und/oder Schwefel, wobei als Aryl- oder Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

außerdem für geradkettiges oder verzweigtes Halogenalkyl mit 1 bis 8 Kohlenstoffatomen und 1 bis 17 gleichen oder verschiedenen Halogenatomen - insbesondere Fluor, Chlor und/oder Brom - steht,

außerdem für gegebenenfalls einfach bis zweifach, gleich oder verschieden

substituiertes, geradkettiges oder verzweigtes Alkenyl mit 2 bis 8 Kohlenstoffatomen steht, wobei als Substituenten infrage kommen:
Cyano oder gegebenenfalls einfach bis dreifach, gleich oder verschieden substituiertes Aryl mit 6 oder 10 Kohlenstoffatomen, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für geradkettiges oder verzweigtes Halogenalkenyl mit 2 bis 8 Kohlenstoffatomen und 1 bis 13 gleichen oder verschiedenen Halogenatomen - insbesondere Fluor, Chlor und/oder Brom - steht,
außerdem für geradkettiges oder verzweigtes Alkinyl mit 2 bis 8 Kohlenstoffatomen steht,
außerdem für geradkettiges oder verzweigtes Halogenalkinyl mit 2 bis 8 Kohlenstoffatomen und 1 bis 13 gleichen oder verschiedenen Halogenatomen - insbesondere Fluor, Chlor und/oder Brom - steht,
außerdem für jeweils gegebenenfalls einfach bis vierfach, gleich oder verschieden substituiertes Cycloalkyl oder Cycloalkenyl mit jeweils 3 bis 7 Kohlenstoffatomen steht, wobei als Substituenten jeweils infrage kommen:
Fluor, Chlor, Brom, Iod sowie jeweils geradkettiges oder verzweigtes Alkyl oder Halogenalkyl mit jeweils 1 bis 6 Kohlenstoffatomen und gegebenenfalls 1 bis 13 gleichen oder verschiedenen Halogenatomen;
außerdem für jeweils gegebenenfalls einfach bis fünffach, gleich oder verschieden substituiertes Aryl mit 6 oder 10 Kohlenstoffatomen oder Heterocyclyl mit 2 bis 9 Kohlenstoffatomen und 1 bis 4 gleichen oder verschiedenen Heteroatomen, insbesondere Stickstoff, Sauerstoff und/oder Schwefel steht, wobei als Aryl- bzw. Heterocyclylsubstituenten jeweils infrage kommen:
Halogen, Cyano, Nitro, jeweils geradkettiges oder vertweigtes Alkyl, Alkoxy oder Alkythio mit jeweils 1 bis 4 Kohlenstoffatomen, jeweils geradkettiges oder verzweigtes Halogenalkyl, Halogenalkoxy, Halogenalkythio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis dreifach, gleich oder verschieden durch Halogen und/oder geradkettiges oder verzweigtes Alkyl mit 1 bis 4 Kohlenstoffatomen substituiertes Phenyl;
und außerdem für einen Rest der Formel

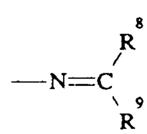

steht und

X für Sauerstoff oder Schwefel steht, wobei

$R^8$ für Wasserstoff oder Alkyl mit 1 bis 6 Kohlenstoffatomen steht und

$R^9$ für Alkyl mit 1 bis 6 Kohlenstoffatomen oder für gegebenenfalls einfach bis dreifach, gleich oder verschieden substituiertes Phenyl steht, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl

oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl oder

$R^8$ und $R^9$ gemeinsam mit dem Kohlenstoffatom, an welches sie gebunden sind, für einen Cycloalkylrest mit 3 bis 7 Kohlenstoffatomen stehen.

Insbesondere bevorzugt sind Verbindungen der Formel (I), bei welchen

$R^1$, $R^2$, $R^3$, $R^4$, $R^6$ und $R^7$ für Wasserstoff, Fluor, Chlor oder Brom, für geradkettiges oder verzweigtes Alkyl mit 1 bis 4 Kohlenstoffatomen, Halogenalkyl mit 1 bis 4 Kohlenstoffatomen und 1 bis 9 gleichen oder verschiedenen Halogenatomen, Cycloalkyl mit 3 bis 6 Kohlenstoffatomen oder für gegebenenfalls einfach oder zweifach, gleich oder verschieden substituiertes Phenyl stehen, wobei als Substituenten infrage kommen:

Halogen, Cyano, Nitro, jeweils geradkettiges oder verzweigtes Alkyl, Alkoxy oder Alkylthio mit jeweils 1 bis 3 Kohlenstoffatomen, jeweils geradkettiges oder verzweigtes Halogenalkyl, Halogenalkoxy, Halogenalkylthio mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach oder zweifach, gleich oder verschieden durch Halogen und/oder geradkettiges oder verzweigtes Alkyl mit 1 bis 3 Kohlenstoffatomen substituiertes Phenyl;

$R^5$ für Wasserstoff oder für ein Äquivalent eines Alkali-, Erdalkali- oder eines gegebenenfalls einfach bis vierfach, gleich oder verschieden durch geradkettiges oder verzweigtes Alkyl mit 1 bis 12 Kohlenstoffatomen und/oder Benzyl substituierten Ammoniumkations steht;

außerdem für gegebenenfalls ein- bis dreifach, gleich oder verschieden substituiertes, geradkettiges oder verzweigtes <u>Alkyl</u> mit 1 bis 12 Kohlenstoffatomen steht, wobei als Substituenten infrage kommen:

Hydroxy, Cyano, jeweils geradkettiges oder verzweigtes Alkoxy, Alkoxycarbonyl, Alkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 6 Kohlenstoffatomen in den einzelnen Alkylteilen, jeweils geradkettiges oder verzweigtes Halogenalkoxy oder Halogenalkylthio mit jeweils 1 bis 6 Kohlenstoffatomen und 1 bis 13 Halogenatomen sowie jeweils gegebenenfalls im Phenyl- oder Heteroarylteil einfach bis dreifach, gleich oder verschieden substituiertes Phenyl, Phenoxy, Phenylthio, Phenylamino, Phenylcarbonyl, Phenylsulfonyl, Pyridyl, Furanyl, Tetrahydrofuranyl, Thienyl oder Thiazolyl, wobei als Phenyl- oder Heteroarylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;

außerdem für geradkettiges oder verzweigtes <u>Halogenalkyl</u> mit 1 bis 8 Kohlenstoffatomen und 1 bis 17 gleichen oder verschiedenen Halogenatomen - insbesondere Fluor, Chlor und/oder Brom - steht,

außerdem für geradkettiges oder verzweigtes <u>Alkenyl</u> mit 2 bis 6 Kohlenstoffatomen steht;

außerdem für geradkettiges oder verzweigtes <u>Halogenalkenyl</u> mit 2 bis 8 Kohlenstoffatomen und 1 bis 15 gleichen oder verschiedenen Halogenatomen - insbesondere Fluor, Chlor und/oder Brom - steht,

außerdem für geradkettiges oder verzweigtes <u>Alkinyl</u> mit 2 bis 6 Kohlenstoffatomen steht ;

außerdem für geradkettiges oder verzweigtes <u>Halogenalkinyl</u> mit 2 bis 8 Kohlenstoffatomen und 1 bis 13 gleichen oder verschiedenen Halogenatomen - ins-besondere Fluor, Chlor und/oder Brom - steht,

außerdem für jeweils gegebenenfalls ein- bis dreifach, gleich oder verschieden durch Fluor, Chlor und/oder Methyl substituiertes Cyclopropyl oder Cyclohexyl steht;
außerdem für gegebenenfalls ein- bis dreifach, gleich oder verschieden substituiertes Phenyl steht, wobei als Substituenten jeweils infrage kommen:
Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für jeweils gegebenenfalls ein- bis dreifach, gleich oder verschieden substituiertes Pyridyl, Furanyl, Tetrahydrofuranyl, Thienyl oder Thiazolyl steht, wobei als Substituenten jeweils infrage kommen:
jeweils geradkettiges oder verzweigtes Alkyl oder Alkoxy mit jeweils 1 bis 4 Kohlenstoffatomen;
und außerdem für einen Rest der Formel

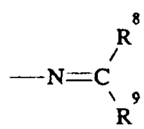

steht und

X für Sauerstoff oder Schwefel steht, wobei

$R^8$ für Wasserstoff oder Alkyl mit 1 bis 4 Kohlenstoffatomen steht und

$R^9$ für Alkyl mit 1 bis 4 Kohlenstoffatomen oder für gegebenenfalls einfach bis dreifach, gleich oder verschieden substituiertes Phenyl steht, wobei als Substituenten infrage kommen:
Halogen, Cyano, Nitro, jeweils geradkettiges oder verzweigtes Alkyl, Alkoxy oder Alkylthio mit jeweils 1 bis 3 Kohlenstoffatomen, jeweils geradkettiges oder verzweigtes Halogenalkyl, Halogenalkoxy, Halogenalkylthio mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach oder zweifach, gleich oder verschieden durch Fluor und/oder Chlor und/oder geradkettiges oder verzweigtes Alkyl mit 1 bis 3 Kohlenstoffatomen substituiertes Phenyl oder

$R^8$ und $R^9$ gemeinsam mit dem Kohlenstoffatom, an welches sie gebunden sind, für einen Cycloalkylrest mit 5 oder 6 Kohlenstoffatomen stehen.

Ganz besonders bevorzugt sind Verbindungen der Formel (I), bei welchen

$R^1$, $R^2$, $R^3$ und $R^4$ für Wasserstoff stehen,

$R^5$ für Wasserstoff, für ein Natrium-, Kalium-, Calcium- oder für ein gegebenenfalls ein- bis vierfach, gleich oder verschieden durch Methyl, Ethyl, n- oder i-Propyl, n-, i-, s- oder t-Butyl, n- oder i-Pentyl, n- oder i-Hexyl, n- oder i-Dodecyl und/oder Benzyl substituiertes Ammoniumion steht;
außerdem für Methyl, Ethyl, n- oder i-Propyl, n-, i-, s- oder t-Butyl n- oder i-Pentyl, n- oder i-Hexyl, Fluormethyl, Trifluormethyl, Hydroxyethyl, Cyanoethyl, Methoxyethyl, Methylthioethyl, Methylaminoethyl, Ethylaminoethyl, N,N-Dimethylaminoethyl, N,N-Diethylaminoethyl, Methoxycarbonylmethyl, Ethoxycarbonylmethyl, n- oder i-Propoxycarbonylmethyl, Methoxycarbonylethyl, Ethoxycarbonylethvl, n- oder i-Propoxycarbonylethyl, Methylcarbonylmethyl, Methylcarbonylethyl, N-Methylaminocarbonylmethyl, N-Methylaminocarbonylethyl, N-Ethylaminocarbonylmethyl, N-Ethylaminocarbonylethyl, N,N-Dimethylaminocarbonylmethyl N,N-Diethylaminocarbonylmethyl, N,N-Dimethylaminocarbonylethyl, N,N-Diethylaminocarbonylethyl, N-Acetylaminomethyl, N-Acetylaminoethyl, N-Propionylaminomethyl, N-Propionylaminoethyl, Methylsulfonylmethyl, Methylsulfonylethyl, Ethylsulfonylmethyl, Ethylsulfonylethyl, Allyl, Propargyl, Cyclopropyl, Cyclopentyl, Cyclohexyl oder für jeweils gegebenenfalls ein- bis dreifach, gleich oder verschieden substituiertes Phenyl, Pyridyl, Benzyl oder Furanylmethyl steht, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy oder Trifluormethylthio,

und außerdem für einen Rest der Formel

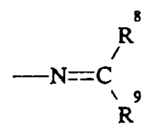

steht

$R^6$ für Methyl steht,

$R^7$ für Isopropyl steht und

X für Sauerstoff steht, wobei

$R^8$ für Wasserstoff, Methyl oder Ethyl steht und

$R^9$ für Methyl, Ethyl oder Phenyl steht.

Im einzelnen seien die bei den Herstellungsbeispielen genannten Verbindungen der allgemeinen Formel (I) genannt.

Verwendet man beispielsweise Pyrrolo-[1,2-b]-pyridazin-2,3-dicarbonsäurediethylester und 2-Amino-2,3-dimethylbuttersäureamid als Ausgangsverbindungen, so läßt sich der Reaktionsablauf des erfindungsgemäßen Verfahrens (a) durch das folgende Formelschema darstellen:

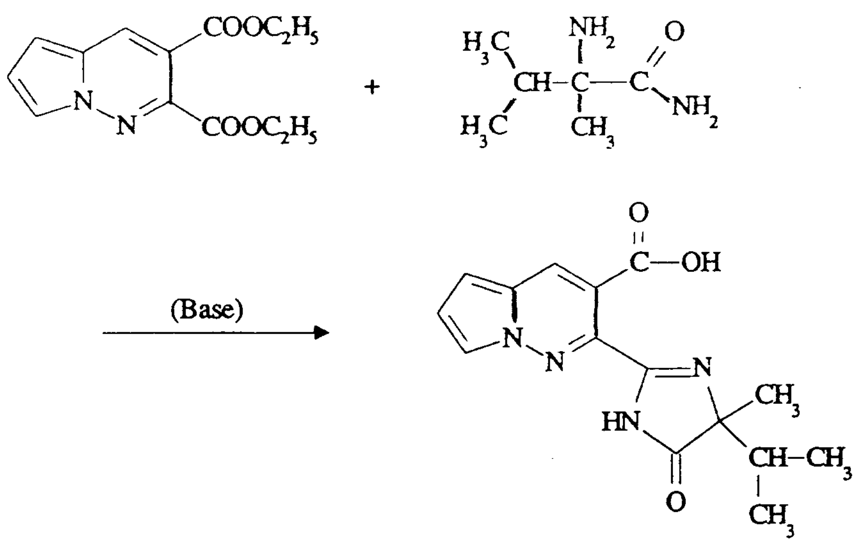


Verwendet man beispielsweise 2-(5-Methyl-5-isopropyl-3H-imidazolin-4-on-2-yl)-pyrrolo-[1,2-b]-pyridazin-3-carbonsäure und Ethanol als Ausgangsstoffe sowie N,N'-Dicyclohexylcarbodiimid als Kondensationsmittel, so läßt sich der Reaktionsablauf des erfindungsgemäßen Verfahrens (b) durch das folgende Formelschema darstellen:

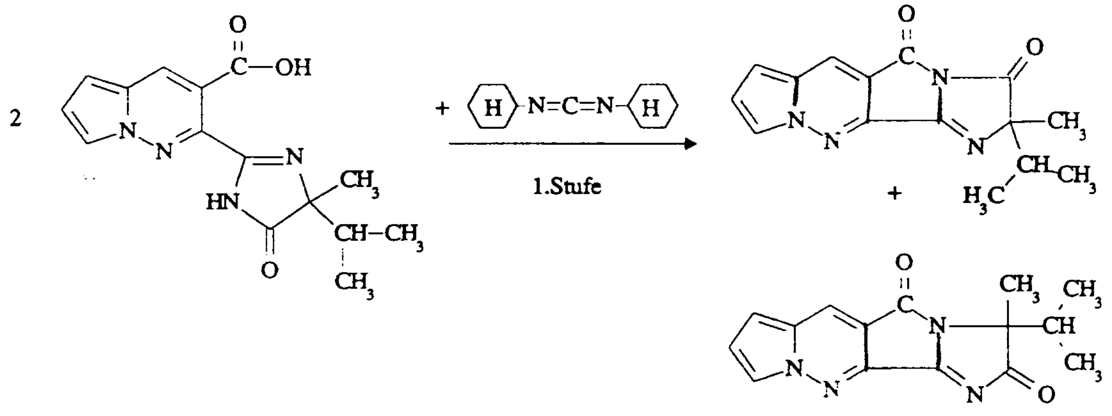


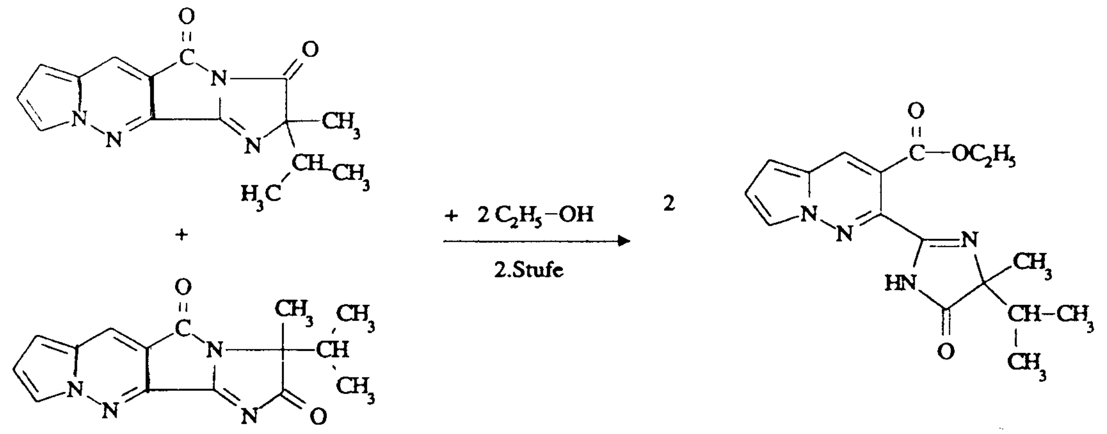


Die zur Durchführung des erfindungsgemaßen Verfahrens (a) als Ausgangsstoffe benötigten Pyrrolopyridazin-dicarbonsäurediester sind durch die Formel (II) allgemein definiert. In dieser Formel (II) stehen $R^1$, $R^2$, $R^3$ und $R^4$ vorzugsweise für diejenigen Reste, die bereits im Zusammenhang mit der Beschreibung der erfindungsgemäßen Verbindungen der Formel (I) als bevorzugt für diese Substituenten genannt wurden. $R^{10}$ und $R^{11}$ stehen vorzugsweise unabhängig voneinander jeweils für geradkettiges oder verzweigtes Alkyl mit 1 bis 4 Kohlenstoffatomen, insbesondere für Methyl oder Ethyl.

Die Pyrrolopyridazin-dicarbonsäurediester der Formel (II) sind noch nicht bekannt und ebenfalls Gegenstand der Erfindung. Man erhält sie; wenn man N-Aminopyrrole der Formel (VI),

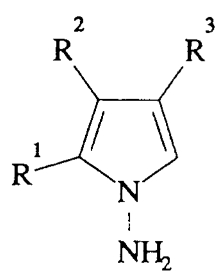

(VI)

in welcher

$R^1$, $R^2$ und $R^3$ die oben angegebene Bedeutung haben,

zunächst in einer ersten Stufe mit Oxalylessigester-Derivaten der Formel (VII),

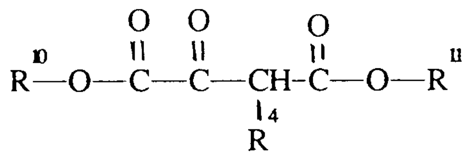

(VII)

in welcher

$R^4$, $R^{10}$ und $R^{11}$ die oben angegebenen Bedeutungen haben,

gegebenenfalls in Gegenwart eines Verdünnungsmittels wie beispielsweise Ethanol bei Temperaturen zwischen 30 °C und 80 °C umsetzt und anschließend die so erhältlichen Aminopyrrol-Addukte der Formel (VIII),

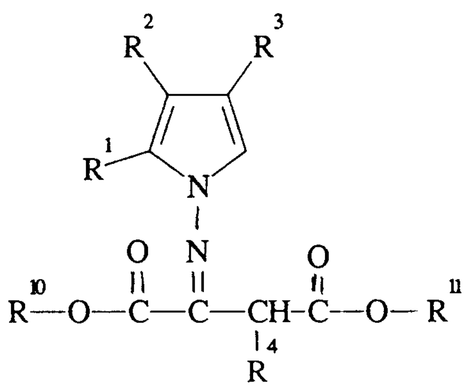

(VIII)

in welcher

$R^1$, $R^2$ ,$R^3$, $R^4$, $R^{10}$ und $R^{11}$ die oben angegebenen Bedeutungen haben,

in einer anschließenden zweiten Stufe mit Phosphoroxychlorid/Dimethylformamid gegebenenfalls in Gegenwart eines Verdünnungsmittels wie beispielsweise Hexan oder auch ohne Zusatz eines weiteren Verdünnungsmittels bei Temperaturen zwischen - 20 °C und 80 °C cyclisiert.

N-Aminopyrrole der Formel (VI) sind bekannt oder erhältlich in Analogie zu bekannten Verfahren (vergl. z.B. Tetrahedron Lett. 29, 4823-4826 [1988]; Chem. Ber. 102, 3268-3276 [1969]).

Oxalylessigester-Derivaten der Formel (VII) sind ebenfalls bekannt oder erhältlich in Analogie zu bekannten Verfahren (vergl. z.B. J. Org. Chem. 56, 7360 [1991]; J. Heterocycl. Chem. 27, 1857-1860 [1990]; J. Heterocycl. Chem. 27, 579-582 [1990]; J. Heterocycl. Chem. 20, 623-628 [1983]; Synth. Commun. 9, 603-607 [1979]).

Aminopyrrol-Addukte der Formel (VIII) sind noch nicht bekannt und ebenfalls Gegenstand der Erfindung.

Die zur Durchführung des erfindungsgemäßen Verfahren (a) weiterhin als Edukte erforderlichen Aminosäureamide sind durch die Formel (III) allgemein definiert. In dieser Formel (III) stehen $R^6$ und $R^7$ vorzugsweise für diejenigen Reste, die bereits im Zusammenhang mit der Beschreibung der erfindungsgemäßen Stoffe der Formel (I) als bevorzugt für diese Substituenten genannt wurden.

Die Aminosäureamide der Formel (III) sind allgemein bekannt oder erhältlich in Analogie zu allgemein bekannten Verfahren (vergl. z.B. Houben-Weyl-Müller "Methoden der organischen Chemie", Thieme Verlag Stuttgart 1974; Band XV/1, S.46ff; Band XV/2, S.112ff).

Die zur Durchführung des erfindungsgemäßen Verfahrens (b) als Edukte erforderlichen Imidazolinyl-pyrrolo-pyridazin-carbonsäuren sind durch die Formel (Ia) allgemein definiert. In dieser Formel (Ia) stehen $R^1$, $R^2$, $R^3$, $R^4$, $R^6$ und $R^7$ vorzugsweise für diejenigen Reste, die bereits im Zusammenhang mit der Beschreibung der erfindungsgemäßen Stoffe der Formel (I) als bevorzugt für diese Substituenten genannt wurden.

Die Imidazolinyl-pyrrolo-pyridazin-carbonsäuren der Formel (Ia) sind erfindungsgemäße Verbindungen und erhältlich mit Hilfe des erfindungsgemäßen Verfahren (a).

Die zur Durchführung des erfindungsgemäßen Verfahrens (b) weiterhin als Edukte erforderlichen Alkohole, Thiole und Oxime sind durch die Formel (V) allgemein definiert. In dieser Formel (V) stehen $R^5$

und X vorzugsweise für diejenigen Reste, die bereits im Zusammenhang mit der Beschreibung der erfindungsgemäßen Stoffe der Formel (I) als bevorzugt für diese Substituenten genannt wurden.

Die Alkohole, Thiole und Oxime der Formel (V) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichiorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (a) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 180°C, vorzugsweise bei Temperaturen zwischen 20°C und 120°C.

Das erfindungsgemäße Verfahren (a) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (a) setzt man pro Mol Pyrrolopyridazin-dicarbonsäurediester der Formel (II) im allgemeinen 1,0 bis 1,5 Mol, vorzugsweise äquimolare Mengen Aminosäureamid der Formel (III) und 1,0 bis 5,0 Mol, vorzugsweise 1,0 bis 3,0 Mol Base als Reaktionshilfsmittel ein. Die Reaktionsdurchführung, Aufarbeitung und Isolierung der Reaktionsprodukte erfolgt nach bekannten Verfahren (vergl. hierzu beispielsweise EP 41623 oder die Herstellungsbeispiele).

Die 1. Stufe des erfindungsgemaßen Verfahrens (b) wird in Gegenwart eines geeigneten Kondensationsmittels durchgeführt. Als solche kommen alle für derartige Cyclisierungsreaktionen üblichen Kondensationsmittel infrage. Beispielhaft genannt seien Säurehalogenidbildner, wie Phosphortribromid, Phosphorpentachlorid, Phosphoroxychlorid oder Thionylchlorid, Anhydridbildner, wie Chlorameisensäureethylester oder Methansulfonylchlorid, Carbodiimide, wie N,N'-Dicyclohexylcarbodiimid (DCC) oder N,N'-Diisopropylcarbodiimid oder andere übliche Kondensationsmittel, wie N,N'-Carbonyldiimidazol, Triphenylphosphin/Tetrachlorkohlenstoff oder 2-Ethoxy-N-ethoxycarbonyl-1,2-dihydrochinolin (EEDQ).

Als Verdünnungsmittel zur Durchführung der 1. Stufe des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid oder Alkohole, wie Methanol, Ethanol oder Isopropanol.

Die 1. Stufe des erfindungsgemäßen Verfahrens (b) kann gegebenenfalls in Gegenwart eines geeigneten Reaktionshilfsmittels durchgeführt werden. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 1. Stufe des erfindungsgemäßen Verfahrens (b) in einem größeren Bereich variiert werden. Im allgemeinen arbeitet man bei Temperaturen

zwischen -20°C und 100°C, vorzugsweise bei Temperaturen zwischen 0°C und 80°C.

Zur Durchführung der 1. Stufe des erfindungsgemäßen Verfahrens (b) setzt man pro Mol Imidazolinyl-pyrrolo-pyridazin-carbonsäure der Formel (Ia) im allgemeinen 1,0 bis 5,0 Mol, vorzugsweise 1,0 bis 1,5 Mol Kondensationsmittel und gegebenenfalls 0,001 bis 2,0 Mol, vorzugsweise 0,1 bis 1,2 Mol Base als Reaktionshilfsmittel ein. Die Reaktionsdurchführung, Aufarbeitung und Isolierung der Reaktionsprodukte erfolgt nach bekannten Verfahren (vergl. hierzu beispielsweise EP 41623 oder die Herstellungsbeispiele).

Als Verdünnungsmittel zur Durchführung der 2. Stufe des erfindungsgemäßen Verfahrens (b) kommen ebenfalls inerte organische Lösungsmittel infrage. Vorzugsweise verwendet man die bei der 1. Stufe des erfindungsgemäßen Verfahrens (b) genannten Verdünnungsmittel. Es ist jedoch auch möglich, bei Verwendung von flüssigen Alkoholen, Thiolen oder Oximen der Formel (V) als Reaktionskomponente, diese in einem entsprechenden Überschuß gleichzeitig als Verdünnungsmittel einzusetzen

Die 2. Stufe des erfindungsgemäßen Verfahrens (b) kann gegebenenfalls in Gegenwart eines geeigneten basischen Reaktionshilfsmittels durchgeführt werden. Als solche kommen alle üblichen anorganischen oder organischen Basen infrage. Hierzu gehören beispielsweise Erdalkali- oder Alkalimetallhydroxide, wie Natriumhydroxid. Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 2. Stufe des erfindungsgemäßen Verfahrens (b) in einem größeren Bereich variiert werden. Im allgemeinen arbeitet man bei Temperaturen zwischen 0°C und 150°C, vorzugsweise bei Temperaturen zwischen 20°C und 100°C.

Die 2. Stufe des erfindungsgemäßen Verfahrens (b) wird üblicherweise unter Normaldruck durchgeführt.

Zur Durchführung der 2. Stufe des erfindungsgemäßen Verfahrens (b) setzt man pro Mol Imidazo-pyrrolo-pyridazine der Formeln (IVa) bzw. (IVb) im allgemeinen 1,0 bis 50 Mol, vorzugsweise 1,0 bis 10 Mol Alkohol, Thiol oder Oxim der Formel (V) und gegebenenfalls 0,1 bis 2,0 Mol, vorzugsweise 0,5 bis 1,2 Mol Base als Reaktionshilfsmittel ein.

Die Reaktionsdurchführung, Aufarbeitung und Isolierung der Reaktionsprodukte erfolgt nach bekannten Verfahren (vergl. hierzu beispielsweise EP 41623 oder die Herstellungsbeispiele).

In einer besonderen Durchführungsform ist es auch möglich, die 1. und die 2. Stufe des erfindungsgemäßen Verfahrens (b) in einem Reaktionschritt in einem sogenannten "Eintopfverfahren" durchzuführen. Man geht dabei aus von Imidazolinyl-pyrrolo-pyridazin-carbonsäure der Formel (Ia) und setzt diese nacheinander im "Eintopfverfahren" zunächst mit einem Kondensationsmittel und anschließend mit einem Alkohol, Thiol oder Oxim der Formel (V) um.

Die Reinigung der Endprodukte der Formel (I) erfolgt mit Hilfe üblicher Verfahren, beispielsweise durch Säulenchromatographie oder durch Umkristallisieren.

Erfindungsgemäße Verbindungen der Formel (I), bei welchen $R^5$ für Wasserstoff steht, können mit Hilfe üblicher Methoden in Salze überführt werden, beispielsweise indem man sie in einem geeigneten inerten Lösungsmittel löst, anschließend eine entsprechende Base zugibt und das Salz durch Abfiltrieren oder Abdestillieren des Lösungsmittels isoliert und gegebenenfalls durch Waschen mit einem inerten Lösungsmittel oder durch Umkristallisieren reinigt.

Die Charakterisierung der Verbindungen erfolgt mit Hilfe des Schmelzpunktes oder bei nicht kristallisierenden Verbindungen mit Hilfe der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alope-

curus, Apera.

Monokotyle Kulturen der Gattungen: Oryza, Zea, Triticum, Hordeum, Avena, Secale, Sorghum, Panicum, Sacchatum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von dikotylen Unkräutern in monokotylen Kulturen wie beispielsweise Weizen einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Noflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr,

Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuronethyl, Chlorsulfuron, Cinosulfuron, Metsulfuron-methyl, Nicosulfuron, Primisulfuron, Pyrazosulfuron-ethyl, Thifensulfuronmethyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminothazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anweildung geschieht in üblicher Weise. z.B. durch Gießen, Spritzen; Sprühen, Streuen.

Die erfindungsgemäßen Wirkstoffe können sowohl vor, als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro Hektar.

Die Herstellung und die Verwendung der erfindungsgemäßen Wirkstoffe geht aus den nachfolgenden Beispielen hervor.

Herstellungsbeispiele:

Beipiel 1:

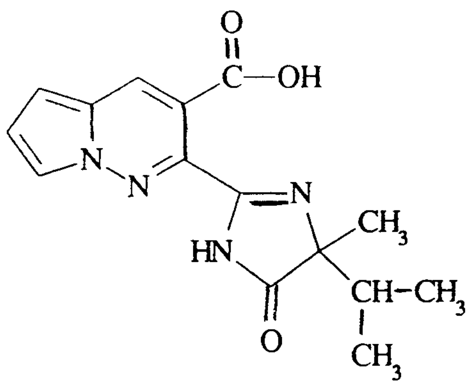

(Verfahren a)

Zu einer Mischung von 15 g (0,057 Mol) Pyrrolo-[1,2-b]-pyridazin-2,3-dicarbonsäurediethylester und 7,44 g (0,057 Mol) 2-Amino-2,3-dimethylbuttersäureamid in 400 ml wasserfreiem Toluol gibt man portionsweise 18,4 g (0,125 Mol) Kalium-tert-butylat und rührt anschließend 2 Stunden bei 80°C. Zur Aufarbeitung wird die Reaktionsmischung abgekühlt, der ausgefallene Feststoff abfiltriert, mehrfach mit Diethylether nachgewaschen und anschließend in Wasser gelöst. Die wässrige Lösung wird mit halbkonzentrierter Salzsäure auf pH 5 gebracht und fünfmal mit jeweils 100 ml Dichlonmethan extrahiert. Die vereinigten organischen Extrakte werden über Natriumsulfat getrocknet und im Vakuum eingeengt. Der Rückstand läßt sich durch Chromatographie an Kieselgel reinigen.

Man erhält 9,2 g (51 % der Theorie) 2-(5-Methyl-5-isopropyl-3H-imidazolin-4-on-2-yl)-pyrrolo-[1,2-b]-pyridazin-3-carbonsäure, Schmelzpunkt 215 ° C (Zers.).

Herstellunge der Ausgangsverbindung:

Beispiel II-1:

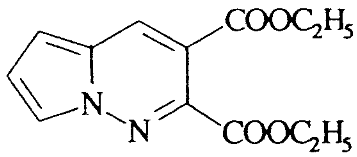

Zu 75,4 g (0,29 Mol) 1-[(1,2-Bis-(ethoxycarbonyl)-ethyliden)-imino]-pyrrol in 90 g (1,23 Mol) Dimethylformamid, gibt man tropfenweise unter Rühren und Eiskühlung bei 15 °C bis 20 °C innerhalb von 30 Minuten 46 g (0,29 Mol) Phosphoroxychlorid und rührt nach beendeter Zugabe weitere 30 Minuten bei 20 °C nach. Zur Aufarbeitung wird die Reaktionsmischung in Eiswasser gegossen, mit 4N Natronlauge auf pH 6 gebracht und mit Diethylether extrahiert. die vereinigten Etherphasen werden mit gesättigter wässriger Natriumhydrogencarbonatlösung gewaschen, über Natriumsulfat getrocknet und im Vakuum vom Lösungsmittel befreit Der Rückstand wird durch Chromatographie an Kieselgel (Laufmittel: Petrolether/Essigester 10:1) gereinigt.

Man erhält 27,5 g (36 % der Theorie) Pyrrolo-[1,2-b]-pyridazin-2,3-dicarbonsäurediethylester mit Schmelzpunkt 88-89 °C.

Beispiel VIII-1:

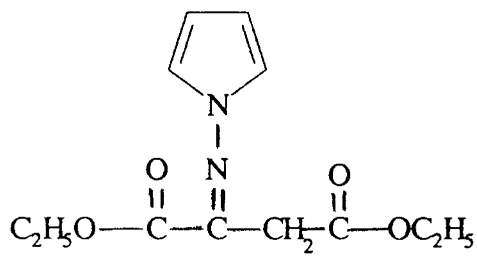

Eine Mischung aus 10 g (0,122 Mol) N-Amino-pyrrol (vergl. z.B. Chem. Ber. 102, 3268-3276 [1969]) und 22,9 g (0,122 Mol) Oxalylessigsäurediethylester (vergl. z.B. Synth. Commun. 9, 603-607 [1979]) in 160 ml Ethanol wird 15 Stunden bei 50 °C gerührt. Zur Aufarbeitung entfernt man flüchtige Bestandteile im Vakuum.

Man erhält 28,3 g (92 % der Theorie) 1 -[(1,2-Bis-(ethoxycarbonyl)-ethyliden)-imino]-pyrrol als Öl.

$^1$H-NMR ($CDCl_3$/Tetramethylsilan): $\delta$ = 1,27 (t, 3H); 1,40 (t, 3H); 3,93 (s, 2H); 4,22 (q, 2H); 4,41 (q, 2H); 6,28 (m, 2H); 6,97 (m, 2H) ppm.

Beispiel 2:

1. Stufe (IV-1):

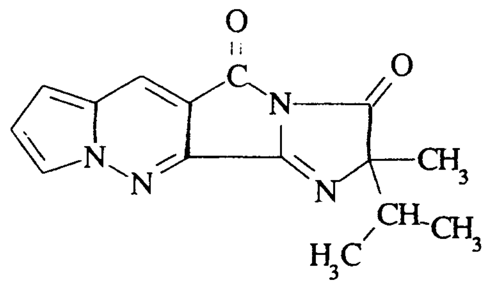

+

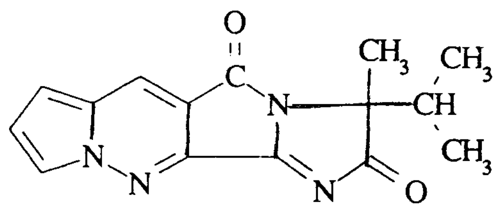

0,6 g (0,002 Mol) 2-(5-Methyl-5-isopropyl-3H-imidazolin-4-on-2-yl)-pyrrolo-[1,2-b]-pyridazin-3-carbonsäure werden in 20 ml Tetrahydrofuran suspendiert, mit 0,25 g (0,002 Mol) N,N'-Diisopropylcarbodiimid versetzt, eine Stunde bei 50°C gerührt, anschließend im Vakuum eingeengt und flüchtige Bestandteile im Hochvakuum entfernt.

Man erhält 0,35 g (62 % der Theorie) einer Isomerenmischung des oben angegebenen Imidazo-pyrrolo-pyridazins mit Schmelzprnnkt 185 °C.

2. Stufe:

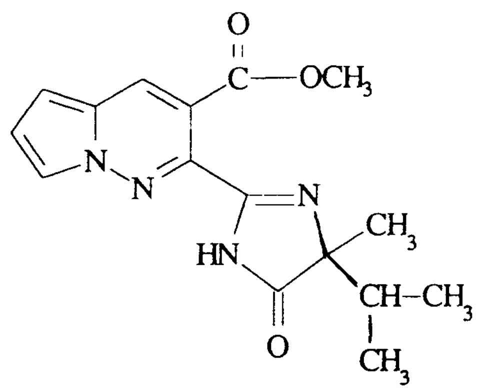

1,41 g (0,0058 Mol) der aus der 1. Stufe erhaltenen Imidazo-pyrrolo-pyridazin-Mischung werden mit 20 ml Methanol und 1 ml Triethylamin versetzt und 24 Stunden auf Siedetemperatur erhitzt. Zur Aufarbeitung wird überschüssiges Methanol im Vakuum abdestilliert, der Rückstand in Essigsäureethylester aufgenommen, mit verdünnter Salzsäure auf pH 4 gebracht, die organische Phase mehrmals mit Wasser gewaschen, über Natriumsulfat getrocknet, im Vakuum eingeengt und der Rückstand durch Chromatographie an Kieselgel (Laufmittel: Petrolether/Essigester 1:1) gereinigt.

Man erhält 1,3 g (83 % der Theorie) 2-(5-Methyl-5-isopropyl-3H-imidazolin-4-on-2-yl)-pyrrolo-[1,2-b]-pyridazin-3-carbonsäuremethylester mit Schmelzpunkt 150 °C.

In entsprechender Weise und gemäß den allgemeinen Angaben zur Herstellung erhält man die folgenden Imidazolinyl-pyrrolo-pyridazine der allgemeinen Formel (I):

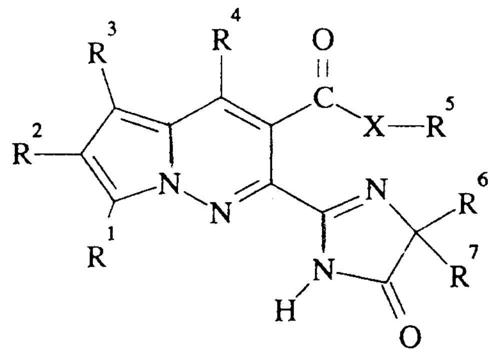

(I)

| Bsp.Nr. | Formel | physikalische Eigenschaften |
|---|---|---|
| 3 | 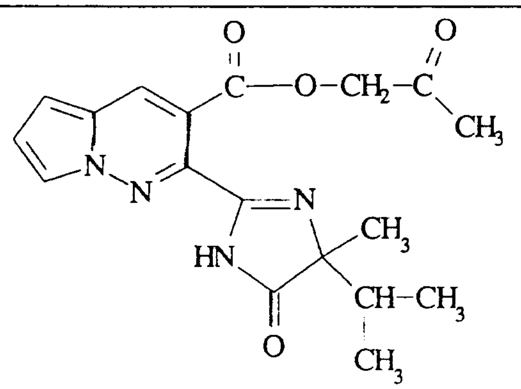 | Fp. 142-144°C |
| 4 | 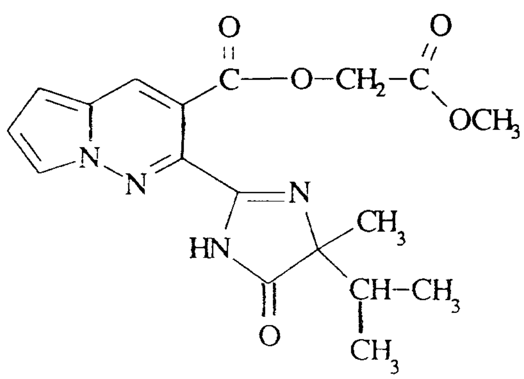 | Fp. 160-162°C |

| Bsp.Nr. | Formel | physikalische Eigenschaften |
|---|---|---|
| 5 | 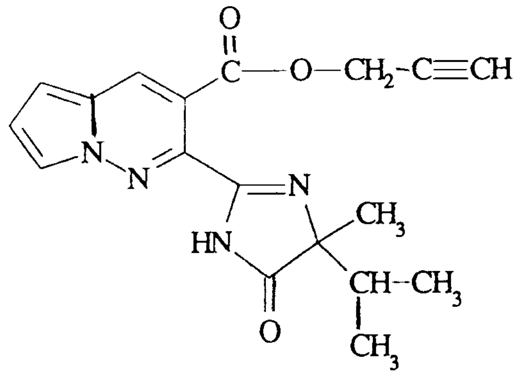 | Fp. 156-158°C |
| 6 | 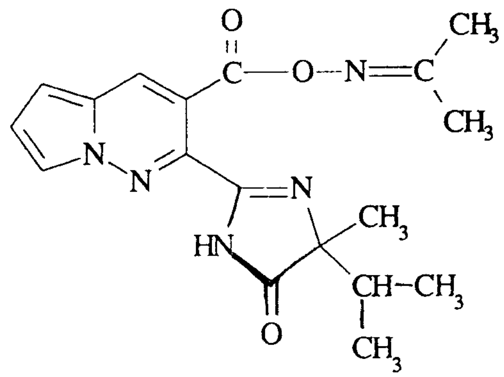 | Fp. 135-136°C |

*) Die $^{1}H$-NMR-Spektren wurden in Deuterochloroform ($CDCl_3$) oder Hexadeutero-Dimethylsulfoxid (DMSO-$d_6$) mit Tetramethylsilan (TMS) als innerem Standard aufgenommen. Angegeben ist die chemische Verschiebung als δ-Wert in ppm.

Anwendungsbeispiele:

In den folgenden Anwendungsbeispielen wurde die nachstehend aufgeführte Verbindung als Vergleichssubstanz eingesetzt:

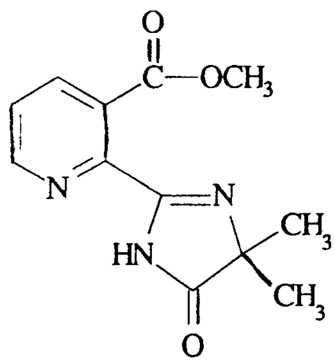

(A)

2-(4,4-Dimethylimidazolin-5-on-2-yl)-pyridin-3-carbonsäuremethylester (bekannt aus EP 41623)

Beispiel A:

**Pre-emergence-Test**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigen in diesem Test z.B. die Verbindungen gemäß den Herstellungsbeispielen 1, 5 und IV-1.

Beipiel B:

**Post-emergence-Test**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschten Wirkstoffmengen pro Flächeneinheit ausgebracht werden. Die Konzentration der Spritzbrühe wird so gewählt, daß in 2000 l Wasser pro Hektar die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100% = totale Vernichtung

Eine deutliche Überlegenheit in der Wirksamkeit bei vergleichbarer Nutzpflanzenselektivität gegenüber dem Stand der Technik zeigen in diesem Test z.B. die Verbindungen gemäß den Herstellungsbeispielen 1, 5, 6 und IV-1.

**Patentansprüche**

1. Imidazolinyl-pyrrolo-pyridazine der allgemeinen Formel (I)

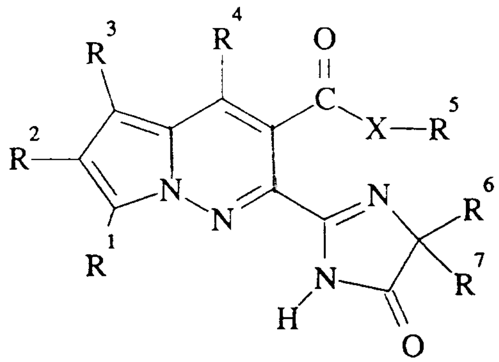

(I)

in welcher

| | |
|---|---|
| $R^1$, $R^2$, $R^3$, $R^4$, $R^6$ und $R^7$ | für Wasserstoff, Halogen, Alkyl, Halogenalkyl, Cycloalkyl oder für gegebenenfalls substituiertes Aryl stehen, |
| $R^5$ | für Wasserstoff, für jeweils gegebenenfalls substituiertes Alkyl, Alkenyl, Alkinyl, Cycloalkyl, Cycloalkenyl, Aryl oder Heterocyclyl, für ein Äquivalent eines anorganischen oder organischen Kations oder für einen Rest der Formel |

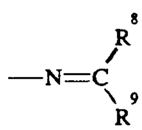

| | |
|---|---|
| | steht und |
| X | für Sauerstoff oder Schwefel steht, wobei |
| $R^8$ | für Wasserstoff oder Alkyl steht und |
| $R^9$ | für Alkyl oder für gegebenenfalls substituiertes Aryl steht oder |
| $R^8$ und $R^9$ | gemeinsam mit dem Kohlenstoffatom , an welches sie gebunden sind, für einen Cycloalkylrest stehen. |

2. Imidazolinyl-pyrrolo-pyridazine der allgemeinen Formel (I) gemäß Anspruch 1, dadurch gekennzeichnet, daß

| | |
|---|---|
| $R^1$, $R^2$, $R^3$, $R^4$, $R^6$ und $R^7$ | für Wasserstoff, Fluor, Chlor, Brom, Iod, für geradkettiges oder verzweigtes Alkyl mit 1 bis 8 Kohlenstoffatomen, Halogenalkyl mit 1 bis 8 Kohlenstoffatomen und 1 bis 17 gleichen oder verschiedenen Halogenatomen, Cycloalkyl mit 3 bis 8 Kohlenstoffatomen oder für gegebenenfalls einfach oder mehrfach, gleich oder verschieden substituiertes Phenyl stehen, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl; |
| $R^5$ | für Wasserstoff oder für ein Äquivalent eines Alkali-, Erdalkali- oder eines gegebenenfalls einfach oder mehrfach, gleich oder verschieden |

durch geradkettiges oder verzweigtes Alkyl mit 1 bis 18 Kohlenstoffatomen und/oder Benzyl substituierten Ammoniumkations steht,
außerdem für gegebenenfalls einfach oder mehrfach, gleich oder verschieden substituiertes, geradkettiges oder verzweigtes Alkyl mit 1 bis 18 Kohlenstoffatomen steht, wobei als Substituenten infrage kommen:
Fluor, Chlor, Brom, Iod, Hydroxy, Cyano, Nitro, Aminocarbonyl, Formamido, jeweils geradkettiges oder verzweigtes Alkoxy, Alkoxycarbonyl, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 8 Kohlenstoffatomen in den einzelnen Alkylteilen und gegebenenfalls 1 bis 17 gleichen oder verschiedenen Halogenatomen, jeweils gegebenenfalls im Aryl- oder Heterocyclylteil einfach oder mehrfach, gleich oder verschieden substituiertes Aryl, Aryloxy, Arylthio, Arylamino, Diarylamino, Arylcarbonyl oder Arylsulfonyl mit jeweils 6 bis 10 Kohlenstoffatomen in den einzelnen Arylteilen oder Heterocyclyl mit 2 bis 9 Kohlenstoffatomen und 1 bis 5 gleichen oder verschiedenen Heteroatomen, insbesondere Stickstoff, Sauerstoff und/oder Schwefel, wobei als Aryl- oder Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
außerdem für gegebenenfalls einfach oder mehrfach, gleich oder verschieden substituiertes, geradkettiges oder verzweigtes Alkenyl mit 2 bis 8 Kohlenstoffatomen steht, wobei als Substituenten infrage kommen:
Fluor, Chlor, Brom, Iod, Cyano oder gegebenenfalls einfach oder mehrfach, gleich oder verschieden substituiertes Aryl mit 6 bis 10 Kohlenstoffatomen, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für gegebenenfalls einfach oder mehrfach, gleich oder verschieden substituiertes, geradkettiges oder verzweigtes Alkinyl mit 2 bis 8 Kohlenstoffatomen steht, wobei als Substituenten infrage kommen:
Fluor, Chlor, Brom oder Iod;
außerdem für jeweils gegebenenfalls einfach oder mehrfach, gleich oder verschieden substituiertes Cycloalkyl oder Cycloalkenyl mit jeweils 3 bis 8 Kohlenstoffatomen steht, wobei als Substituenten jeweils infrage kommen:
Fluor, Chlor, Brom, Iod sowie jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen;
außerdem für jeweils gegebenenfalls einfach oder mehrfach, gleich oder verschieden substituiertes Aryl mit 6 bis 10 Kohlenstoffatomen oder Heterocyclyl mit 2 bis 9 Kohlenstoffatomen und 1 bis 5 gleichen oder verschiedenen Heteroatomen, insbesondere Stickstoff, Sauerstoff und/oder Schwefel steht, wobei als Aryl- bzw. Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
und außerdem für einen Rest der Formel

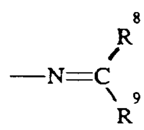

steht und

X für Sauerstoff oder Schwefel steht, wobei

$R^8$ für Wasserstoff oder Alkyl mit 1 bis 8 Kohlenstoffatomen steht und

$R^9$ für Alkyl mit 1 bis 8 Kohlenstoffatomen oder für gegebenenfalls einfach oder mehrfach, gleich oder verschieden substituiertes Phenyl steht, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl oder

$R^8$ und $R^9$ gemeinsam mit dem Kohlenstoffatom, an welches sie gebunden sind, für einen Cycloalkylrest mit 3 bis 8 Kohlenstoffatomen stehen.

**3.** Imidazolinyl-pyrrolo-pyridazine der allgemeinen Formel (I) gemäß Anspruch 1, dadurch gekennzeichnet, daß

$R^1$, $R^2$, $R^3$, $R^4$, $R^6$ und $R^7$ für Wasserstoff, Fluor, Chlor, Brom, für geradkettiges oder verzweigtes Alkyl mit 1 bis 6 Kohlenstoffatomen, Halogenalkyl mit 1 bis 6 Kohlenstoffatomen und 1 bis 13 gleichen oder verschiedenen Halogenatomen, Cycloalkyl mit 3 bis 7 Kohlenstoffatomen oder für gegebenenfalls einfach bis dreifach, gleich oder verschieden substituiertes Phenyl stehen, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenefalls einfach bis dreifach, gleich oder verschieden durch Halogen und/oder geradkettiges oder verzweigtes Alkyl mit 1 bis 4 Kohlenstoffatomen substituiertes Phenyl;

$R^5$ für Wasserstoff oder für ein Äquivalent eines Alkali-, Erdalkali- oder eines gegebenenfalls einfach bis vierfach, gleich oder verschieden

durch geradkettiges oder verzweigtes Alkyl mit 1 bis 16 Kohlenstoffatomen und/oder Benzyl substituierten Ammoniumkations steht,
außerdem für gegebenenfalls einfach bis dreifach, gleich oder verschieden substituiertes, geradkettiges oder verzweigtes Alkyl mit 1 bis 18 Kohlenstoffatomen steht, wobei als Substituenten infrage kommen:
Hydroxy, Cyano, Nitro, Aminocarbonyl, Formamido, jeweils geradkettiges oder verzweigtes Alkoxy, Alkoxycarbonyl, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 6 Kohlenstoffatomen in den einzelnen Alkylteilen und gegebenenfalls 1 bis 13 gleichen oder verschiedenen Halogenatomen, jeweils gegebenenfalls im Aryl- oder Heterocyclylteil einfach bis dreifach, gleich oder verschieden substituiertes Aryl, Aryloxy, Arylthio, Arylamino, Diarylamino, Arylcarbonyl oder Arylsulfonyl mit jeweils 6 oder 10 Kohlenstoffatomen in den einzelnen Arylteilen oder Heterocyclyl mit 2 bis 9 Kohlenstoffatomen und 1 bis 4 gleichen oder verschiedenen Heteroatomen, insbesondere Stickstoff, Sauerstoff und/oder Schwefel, wobei als Aryl- oder Heterocyclylsubstituenten jeweils infrage kommen:
Halogen, Cyano, Nitro, jeweils geradkettiges oder verzweigtes Alkyl. Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;
außerdem für geradkettiges oder verzweigtes Halogenalkyl mit 1 bis 8 Kohlenstoffatomen und 1 bis 17 gleichen oder verschiedenen Halogenatomen - insbesondere Fluor, Chlor und/oder Brom - steht,
außerdem für gegebenenfalls einfach bis zweifach, gleich oder verschieden substituiertes, geradkettiges oder verzweigtes Alkenyl mit 2 bis 8 Kohlenstoffatomen steht, wobei als Substituenten infrage kommen:
Cyano oder gegebenenfalls einfach bis dreifach, gleich oder verschieden substituiertes Aryl mit 6 oder 10 Kohlenstoffatomen, wobei als Substituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für geradkettiges oder verzweigtes Halogenalkenyl mit 2 bis 8 Kohlenstoffatomen und 1 bis 13 gleichen oder verschiedenen Halogenatomen - insbesondere Fluor, Chlor und/oder Brom - steht,
außerdem für geradkettiges oder verzweigtes Alkinyl mit 2 bis 8 Kohlenstoffatomen steht,
außerdem für geradkettiges oder verzweigtes Halogenalkinyl mit 2 bis 8 Kohlenstoffatomen und 1 bis 13 gleichen oder verschiedenen Halogenatomen - insbesondere Fluor, Chlor und/oder Brom - steht,
außerdem für jeweils gegebenenfalls einfach bis vierfach, gleich oder verschieden substituiertes Cycloalkyl oder Cycloalkenyl mit jeweils 3 bis 7 Kohlenstoffatomen steht, wobei als Substituenten jeweils infrage kommen:
Fluor, Chlor, Brom, Iod sowie jeweils geradkettiges oder verzweigtes Alkyl oder Halogenalkyl mit jeweils 1 bis 6 Kohlenstoffatomen und

gegebenenfalls 1 bis 13 gleichen oder verschiedenen Halogenatomen; außerdem für jeweils gegebenenfalls einfach bis fünffach, gleich oder verschieden substituiertes Aryl mit 6 oder 10 Kohlenstoffatomen oder Heterocyclyl mit 2 bis 9 Kohlenstoffatomen und 1 bis 4 gleichen oder verschiedenen Heteroatomen, insbesondere Stickstoff, Sauerstoff und/oder Schwefel steht, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis dreifach, gleich oder verschieden durch Halogen und/oder geradkettiges oder verzweigtes Alkyl mit 1 bis 4 Kohlenstoffatomen substituiertes Phenyl;

und außerdem für einen Rest der Formel

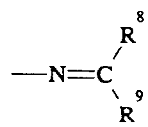

steht und

X für Sauerstoff oder Schwefel steht, wobei

$R^8$ für Wasserstoff oder Alkyl mit 1 bis 6 Kohlenstoffatomen steht und

$R^9$ für Alkyl mit 1 bis 6 Kohlenstoffatomen oder für gegebenenfalls einfach bis dreifach, gleich oder verschieden substituiertes Phenyl steht, wobei als Substituenten infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl oder

$R^8$ und $R^9$ gemeinsam mit dem Kohlenstoffatom, an welches sie gebunden sind, für einen Cycloalkylrest mit 3 bis 7 Kohlenstoffatomen stehen.

**4.** Imidazolinyl-pyrrolo-pyridazine der allgemeinen Formel (I) gemäß Anspruch 1, dadurch gekennzeichnet, daß

$R^1$, $R^2$, $R^3$, $R^4$, $R^6$ und $R^7$ für Wasserstoff, Fluor, Chlor oder Brom, für geradkettiges oder verzweigtes Alkyl mit 1 bis 4 Kohlenstoffatomen, Halogenalkyl mit 1 bis 4 Kohlenstoffatomen und 1 bis 9 gleichen oder verschiedenen Halogenatomen, Cycloalkyl mit 3 bis 6 Kohlenstoffatomen oder für gegebenenfalls einfach oder zweifach, gleich oder verschieden substituiertes Phenyl stehen, wobei als Substituenten infrage kommen:

Halogen, Cyano, Nitro, jeweils geradkettiges oder verzweigtes Alkyl, Alkoxy oder Alkylthio mit jeweils 1 bis 3 Kohlenstoffatomen, jeweils geradkettiges oder verzweigtes Halogenalkyl, Halogenalkoxy, Halogenalkylthio mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3

Kohlenstoffatomen in den einzelnen Alkylteilen sowie gegebenenfalls einfach oder zweifach, gleich oder verschieden durch Halogen und/oder geradkettiges oder verzweigtes Alkyl mit 1 bis 3 Kohlenstoffatomen substituiertes Phenyl;

$R^5$ für Wasserstoff oder für ein Äquivalent eines Alkali-, Erdalkali- oder eines gegebenenfalls einfach bis vierfach, gleich oder verschieden durch geradkettiges oder verzweigtes Alkyl mit 1 bis 12 Kohlenstoffatomen und/oder Benzyl substituierten Ammoniumkations steht;
außerdem für gegebenenfalls ein- bis dreifach, gleich oder verschieden substituiertes, geradkettiges oder verzweigtes Alkyl mit 1 bis 12 Kohlenstoffatomen steht, wobei als Substituenten infrage kommen:
Hydroxy, Cyano, jeweils geradkettiges oder verzweigtes Alkoxy. Alkoxycarbonyl, Alkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 6 Kohlenstoffatomen in den einzelnen Alkylteilen, jeweils geradkettiges oder verzweigtes Halogenalkoxy oder Halogenalkylthio mit jeweils 1 bis 6 Kohlenstoffatomen und 1 bis 13 Halogenatomen sowie jeweils gegebenenfalls im Phenyl- oder Heteroarylteil einfach bis dreifach, gleich oder verschieden substituiertes Phenyl, Phenoxy, Phenylthio, Phenylamino, Phenylcarbonyl, Phenylsulfonyl, Pyridyl, Furanyl, Tetrahydrofuranyl, Thienyl oder Thiazolyl, wobei als Phenyl- oder Heteroarylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für geradkettiges oder verzweigtes Halogenalkyl mit 1 bis 8 Kohlenstoffatomen und 1 bis 17 gleichen oder verschiedenen Halogenatomen - insbesondere Fluor, Chlor und/oder Brom - steht,
außerdem für geradkettiges oder verzweigtes Alkenyl mit 2 bis 6 Kohlenstoffatomen steht ;
außerdem für geradkettiges oder verzweigtes Halogenalkenyl mit 2 bis 8 Kohlenstoffatomen und 1 bis 15 gleichen oder verschiedenen Halogenatomen - insbesondere Fluor, Chlor und/oder Brom - steht,
außerdem für geradkettiges oder verzweigtes Alkinyl mit 2 bis 6 Kohlenstoffatomen steht ;
außerdem für geradkettiges oder verzweigtes Halogenalkinyl mit 2 bis 8 Kohlenstoffatomen und 1 bis 13 gleichen oder verschiedenen Halogenatomen - insbesondere Fluor, Chlor und/oder Brom - steht,
außerdem für jeweils gegebenenfalls ein- bis dreifach, gleich oder verschieden durch Fluor, Chlor und/oder Methyl substituiertes Cyclopropyl oder Cyclohexyl steht;
außerdem für gegebenenfalls ein- bis dreifach, gleich oder verschieden substituiertes Phenyl steht, wobei als Substituenten jeweils infrage kommen:
Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;
außerdem für jeweils gegebenenfalls ein- bis dreifach, gleich oder verschieden substituiertes Pyridyl, Furanyl, Tetrahydrofuranyl, Thienyl oder Thiazolyl steht, wobei als Substituenten jeweils infrage kommen:
jeweils geradkettiges oder verzweigtes Alkyl oder Alkoxy mit jeweils 1 bis 4 Kohlenstoffatomen;
und außerdem für einen Rest der Formel

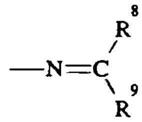

steht und

| | |
|---|---|
| X | für Sauerstoff oder Schwefel steht, wobei |
| $R^8$ | für Wasserstoff oder Alkyl mit 1 bis 4 Kohlenstoffatomen steht und |
| $R^9$ | für Alkyl mit 1 bis 4 Kohlenstoffatomen oder für gegebenenfalls einfach bis dreifach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 3 Kohlenstoffatomen, jeweils geradkettiges oder verzweigtes Halogenalkyl, Halogenalkoxy, Halogenalkylthio mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach oder zweifach, gleich oder verschieden durch Fluor und/oder Chlor und/oder geradkettiges oder verzweigtes Alkyl mit 1 bis 3 Kohlenstoffatomen substituiertes Phenyl oder |
| $R^8$ und $R^9$ | gemeinsam mit dem Kohlenstoffatom, an welches sie gebunden sind, für einen Cycloalkylrest mit 5 oder 6 Kohlenstoffatomen stehen. |

**5.** Verfahren zur Herstellung von Imidazolinyl-pyrrolo-pyridazinen der allgemeinen Formel (I)

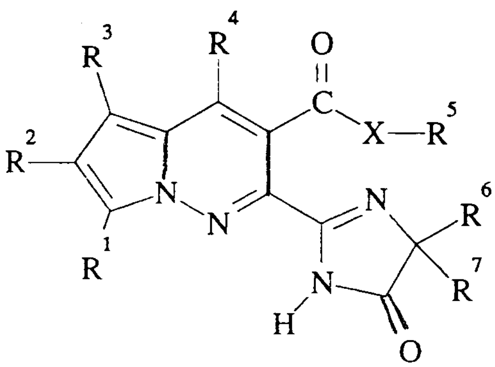

(I)

in welcher

| | |
|---|---|
| $R^1$, $R^2$, $R^3$, $R^4$, $R^6$ und $R^7$ | für Wasserstoff, Halogen, Alkyl, Halogenalkyl, Cycloalkyl oder für gegebenenfalls substituiertes Aryl stehen, |
| $R^5$ | für Wasserstoff, für jeweils gegebenenfalls substituiertes Alkyl, Alkenyl, Alkinyl, Cycloalkyl, Cycloalkenyl, Aryl oder Heterocyclyl, für ein Äquivalent eines anorganischen oder organischen Kations oder für einen Rest der Formel |

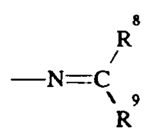

steht und

| | |
|---|---|
| X | für Sauerstoff oder Schwefel steht, wobei |
| $R^8$ | für Wasserstoff oder Alkyl steht und |

$R^9$ für Alkyl oder für gegebenenfalls substituiertes Aryl steht oder
$R^8$ und $R^9$ gemeinsam mit dem Kohlenstoffatom, an welches sie gebunden sind, für einen Cycloalkylrest stehen,

dadurch gekennzeichnet, daß man

(a) Pyrrolopyridazin-dicarbonsäurediester der Formel (II),

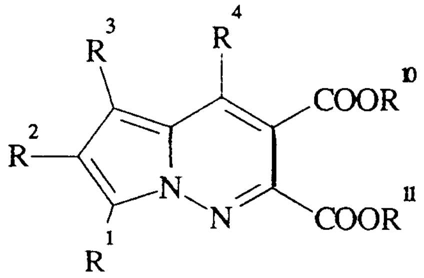

(II)

in welcher
$R^1$, $R^2$, $R^3$ und $R^4$ die oben angegebenen Bedeutungen haben und
$R^{10}$ und $R^{11}$ unabhängig voneinander jeweils für Alkyl stehen.

mit α-Aminosäureamiden der Formel (III),

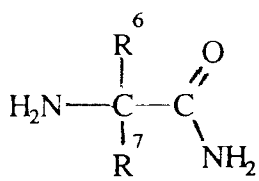

(III)

in welcher
$R^6$ und $R^7$ die oben angegebenen Bedeutungen haben,

gegebenenfalls in Gegenwart eines Verdünnungsmittels und gegebenenfalls in Gegenwart eines Reaktionshilfsmittels umsetzt und gegebenenfalls

(b) in einer anschließenden Folgereaktion die so erhältlichen Imidazolinylpyrrolo-pyridazin-carbonsäuren der Formel (Ia),

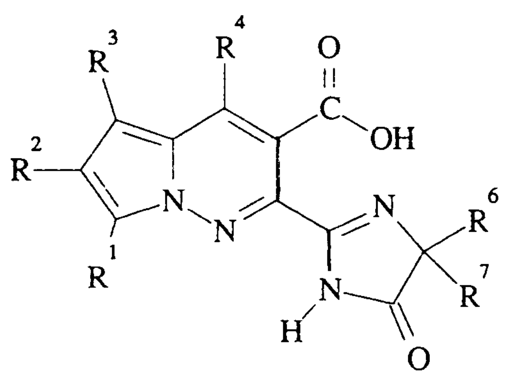

(Ia)

in welcher
$R^1$, $R^2$, $R^3$, $R^4$, $R^6$ und $R^7$ die oben angegebenen Bedeutungen haben,

zunächst in einer 1.Stufe mit einem Kondensationsmittel gegebenenfalls in Gegenwart eines Verdünnungsmittels und gegebenenfalls in Gegenwart eines Reaktionshilfsmittels umsetzt und anschließend die so erhältlichen Imidazo-pyrrolo-pyridazine der Formeln (IVa) und (IVb),

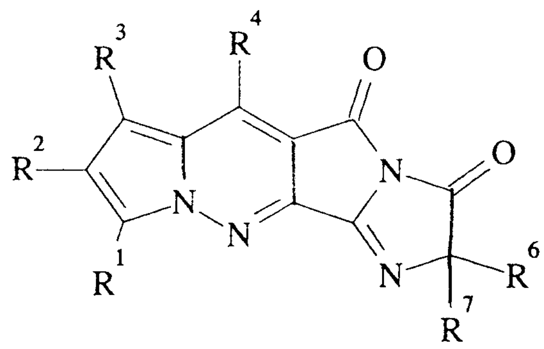

(IVa)

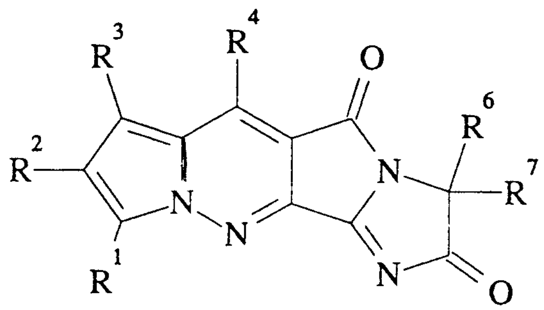

(IVb)

in welcher

$R^1$, $R^2$, $R^3$, $R^4$, $R^6$ und $R^7$ die oben angegebenen Bedeutungen haben,

in einer anschließenden 2. Stufe mit Alkoholen, Thiolen oder Oximen der Formel (V),

$R^5$-X-H (V)

in welcher

$R^5$ und X die oben angegebenen Bedeutungen haben,

gegebenenfalls in Gegenwart eines Verdünnungsmittels und gegebenenfalls in Gegenwart eines Reaktionshilfsmittels umsetzt.

**6.** Herbizide Mittel gekennzeichnet durch einen Gehalt an mindestens einen, Imidazolinyl-pyrrolo-pyridazin der allgemeinen Formel (I) gemäß den Ansprüchen 1 bis 5.

**7.** Verfahren zur Bekämpfung von unerwünschten Pflanzen, dadurch gekennzeichnet, daß man Imidazolinyl-pyrrolo-pyridazine der allgemeinen Formel (I) gemäß den Ansprüchen 1 bis 5 auf Pflanzen und/oder ihren Lebensraum einwirken läßt.

**8.** Verwendung von Imidazolinyl-pyrrolo-pyridazinen der allgemeinen Formel (I) gemäß den Ansprüchen 1 bis 5 zur Bekämpfung von unerwünschten Pflanzen.

**9.** Verfahren zur Herstellung von herbiziden Mitteln, dadurch gekennzeichnet, daß man Imidazolinyl-pyrrolo-pyridazine der allgemeinen Formel (I) gemäß den Ansprüchen 1 bis 5 mit Streckmitteln und/oder oberflächenaktiven Substanzen vermischt

**10.** Pyrrolopyridazin-dicarbonsäureester der allgemeinen Formel (II)

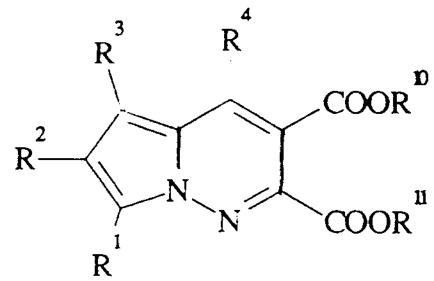

(II)

in welcher

$R^1$, $R^2$, $R^3$ und $R^4$ für Wasserstoff, Halogen, Alkyl, Halogenalkyl, Cycloalkyl oder für gegebenenfalls substituiertes Aryl stehen, und

$R^{10}$ und $R^{11}$ unabhängig voneinander jeweils für Alkyl stehen.

**11.** Aminopyrrol-Addukte der allgemeinen Formel (VIII)

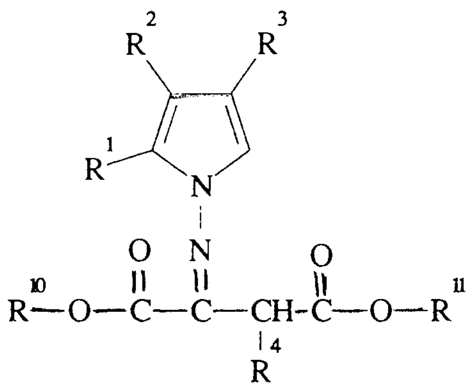

(VIII)

in welcher

$R^1$, $R^2$, $R^3$ und $R^4$ für Wasserstoff, Halogen, Alkyl, Halogenalkyl, Cycloalkyl oder für gegebenenfalls substituiertes Aryl stehen, und

$R^{10}$ und $R^{11}$ unabhängig voneinander jeweils für Alkyl stehen.